# EUROPEAN PATENT APPLICATION

(11) **EP 2 489 313 A1**
(43) Date of publication of application: **22.08.2012**
(21) Application number: 11001343.0
(22) Date of filing: 18.02.2011
(51) Int. Cl.: A61B 17/24, A61B 17/50, A61B 17/221

(54) **Retrieval device for retrieving articles**

(71) Applicant: Krokovay, Anna, 47123 Duisburg (DE)
(72) Inventor: Krokovay, Anna, 47123 Duisburg (DE)

(57) **Abstract**

The invention relates to a retrieval device for retrieving articles comprising: a support member comprising a guide tube and a guide device which is located movably inside the guide tube; and a connecting member comprising a snare; wherein the connecting member and the support member are used together to capture an article or foreign body to be retrieved.

## Description

The present invention preferably relates to the field of medical devices. In particular, the present invention relates to retrieval devices for retrieving articles, e.g. foreign bodies, from a human or veterinary patient. However, the invention is not restricted thereto. The retrieval devices are capable of engaging an article, e.g. a foreign body, at each location of the article, for example at the end or ends of the article or at any other location other than the end or ends of the article.

During medical procedures that utilize catheters, wire guides, cardiac leads, or other medical devices, the device can sometimes break, disconnect, fracture or fragment during use and be left within the patient. In some cases, even years after implantation of a central venous catheter system (e.g. Port-A-Cath), disconnection of the catheter from the reservoir can occur. The detached portion may then travel within the patient's vascular system and come to rest in a luminal organ, vein or artery, and usually at a branching point or in the heart. It can be rather harmful to leave these foreign bodies or articles within the patient, and may result in complications like sepsis, perforation, thrombosis, arrhythmias, myocardial necrosis, or even death. Therefore, it is very important to remove the foreign body or article from the patient.

In WO 2005/081949 A2 a foreign body retrieval device is disclosed which comprises a retriever and a snare. The retriever comprises a hook at one of its ends, wherein the hook comprises a main hook portion and a reverse hook tip portion. Further, the snare includes a snare shaft which is substantially straight along the majority of its length, but also includes a bent portion that links the straight portion of the snare shaft to a loop. The loop is configured to substantially surround a portion of the hook when the snare and the retriever are used together to capture a foreign body. Furthermore, a catheter is provided having a passageway system in which both retriever and snare are slidably positionable. Both the snare and the retriever can be made of Nitinol.

The foreign body retrieval device as disclosed above in WO 2005/081949 A2 has the following disadvantages. One disadvantage is that the snare and the retriever need a lot of space, so that they cannot be used for example in small vessels. In particular, the hook portion of the retriever needs additional space to be turned inside a vessel to capture a foreign body. A further disadvantage is that a catheter has to be provided which is large enough so that the folded snare and the folded hook can be slidably positioned inside the catheter. Outside the catheter the hook portion and the loop have to be deployed. Therefore, the snare and the retriever are made of a shape memory material such as Nitinol. Another disadvantage is that the handling of the foreign body retrieval device is rather uncomfortable for a user. Due to the basically fixedly predetermined form of the hook portion, during intervention it is very difficult for the user to position the hook portion of the retriever at first underneath a foreign body to be retrieved and then to turn and pull the hook portion to capture the foreign body and afterwards to connect the hook portion with the loop. Further, by pushing and pulling the hook portion and the loop inside a vessel, the vessel wall can be injured or even perforated. Besides, after connecting the hook portion with the loop, if the captured foreign body is difficult to mobilize, rather strong forces will be exerted by the loop on the reverse hook tip portion, so that the reverse hook tip portion may easily be caused to bend over. As a result, the hook can slip out of the loop and the foreign body can be lost again, which results in a prolonged intervention. In case of a prolonged and technically complicated procedure under X-ray examination, the patient has to be exposed to a greater amount of X-ray and there is a higher risk of periinterventional complications such as vessel lesions or hemodynamic instability of the patient.

Further, in US 5,562,678 a reversible snare for grasping and retrieving an article such as a cardiac lead from within a human or veterinary patient is disclosed. The snare comprises a retractable closed loop carried by and slidably received in a sheath member. The sheath member comprises an inner dilator sheath slidably received in an inner workstation sheath, which in turn is slidably received in an outer workstation sheath. The closed loop which forms a hook is composed of a shape memory material. In this way the closed loop can be retracted into and extended from the sheath member. Further, the snare comprises a threader which is also carried by and slidably received in the sheath member. The threader is configured as a narrow wire loop and is generally straight. To capture a foreign body or article, the closed loop forming a hook and the threader are manipulated so that the article is completely encircled by the closed loop and the threader, wherein the threader extends through the closed loop. Engagement of the snare and the article is then completed by retracting the threader and the closed loop collapsing the hook about the article and bringing the article into abutment with the distal end on the inner workstation sheath. The inner workstation sheath is then moved outwardly to further retract the engaged article in the outer workstation sheath itself.

The reversible snare as disclosed in US 5,562,678 has also several disadvantages. The sheath member has to be relatively large to encompass the folded or collapsed closed loop, the folded threader and further an article to be retrieved. Moreover, the handling of the reversible snare is very difficult and uncomfortable for a user, since the user has to put the hook underneath the article to be retrieved and then to connect the threader and the hook. By repeated and excessive manipulation inside a vessel, the vessel wall can be injured or even perforated. Further, the hook needs a lot of space inside a vessel, so that the reversible snare can be used only in larger vessels. Besides, if the captured foreign body is difficult to mobilize, the threader can slip out of the closed loop and the foreign body can be lost again. In the case of a prolonged intervention under X-ray examination, the patient has to be exposed to a greater amount of X-ray and there is a higher risk of periinterventional complications such as vessel lesions or hemodynamic instability of the patient. Furthermore, the use of the reversible snare is very restricted. An article to be retrieved by the reversible snare has to be very small and very flexible. Otherwise it is impossible to engage the article in the outer workstation sheath.

The object of the invention is therefore to provide an improved retrieval device for retrieving articles, e.g. foreign bodies.

This object is solved by the retrieval device with the features of claim 1.

According to the invention a retrieval device is provided for retrieving articles, comprising:
a support member comprising a guide tube and a guide device which is located movably inside the guide tube, and
a connecting member comprising a snare,
wherein the connecting member and the support member are used together to capture an article or foreign body to be retrieved.

This has the advantage that an article or foreign body can be easily retrieved by connecting the support member and the connecting member to enclose and retrieve said article or foreign body.

Further embodiments and developments of the invention can be derived from the dependent claims and the description with reference to the figures.

A more detailed understanding of the invention may be had from the following description of preferred embodiments, given by way of example and to be understood in conjunction with the accompanying drawing, wherein:
- Fig. 1: is a schematic view of a retrieval device according to an embodiment of the present invention;
- Fig. 2: is a schematic view of a connecting member of a retrieval device according to an embodiment of the present invention;
- Fig. 3: is a sectional view of a further embodiment of an inventive retrieval device;
- Fig. 4a: is a cross section A-A of Fig. 3;
- Fig. 4b: is a cross section of a retrieval device according to an alternative embodiment of the present invention;
- Fig. 5: shows a step to retrieve an article, e.g., a lost catheter, by the retrieval device of the invention as shown in Fig. 1;
- Fig. 6: shows a further step to retrieve the article shown in Fig. 5 by the retrieval device of the invention as shown in Fig. 1;
- Fig. 7: shows another step to retrieve the article shown in Fig. 6 by the retrieval device of the invention as shown in Fig. 1;
- Fig. 8: shows a further step to retrieve the article shown in Fig. 7 by the retrieval device of the invention as shown in Fig. 1;
- Fig. 9: shows a next step to retrieve the article shown in Fig. 8 by the retrieval device of the invention as shown in Fig. 1;
- Fig. 10: shows a further step to retrieve the article shown in Fig. 9 by the retrieval device of the invention as shown in Fig. 1;
- Fig. 11: shows another step to retrieve the article shown in Fig. 10 by the retrieval device of the invention as shown in Fig. 1;
- Fig. 12: shows still another step to retrieve the article shown in Fig. 11 by the retrieval device of the invention as shown in Fig. 1;
- Fig. 13: is a sectional view of another embodiment of an inventive retrieval device;
- Fig. 14: is a sectional view of a further embodiment of an inventive retrieval device when capturing an article to be retrieved;
- Fig. 15: is a sectional view of still a further embodiment of an inventive retrieval device when the article to be retrieved has been captured and secured by the retrieval device;
- Fig. 16: shows a first example of an article which is lost in the body of a human patient and has to be retrieved;
- Fig. 17: shows the retrieval of the article shown in Fig. 15 by using an inventive retrieval device;
- Fig. 18: shows a second example of an article which is lost in the body of a human patient and has to be retrieved; and
- Fig. 19: shows the retrieval of the article shown in Fig. 17 by using an inventive retrieval device.

In the figures the same reference numbers denote the same or functionally similar components, unless otherwise indicated. Further the illustrations in the figures are schematic and simplified and not drawn to scale.

In the following the retrieval device according to the invention will be explained based on a catheter as an article or foreign body to be retrieved from a human patient. However, the invention is not restricted to a catheter as an article or foreign body to be retrieved from a human or veterinary patient. As described above, an article or foreign body to be retrieved can be for example a wire guide, a cardiac lead, or any other device in a human or veterinary patient or a part thereof which has for example been disconnected, lost or broken off.

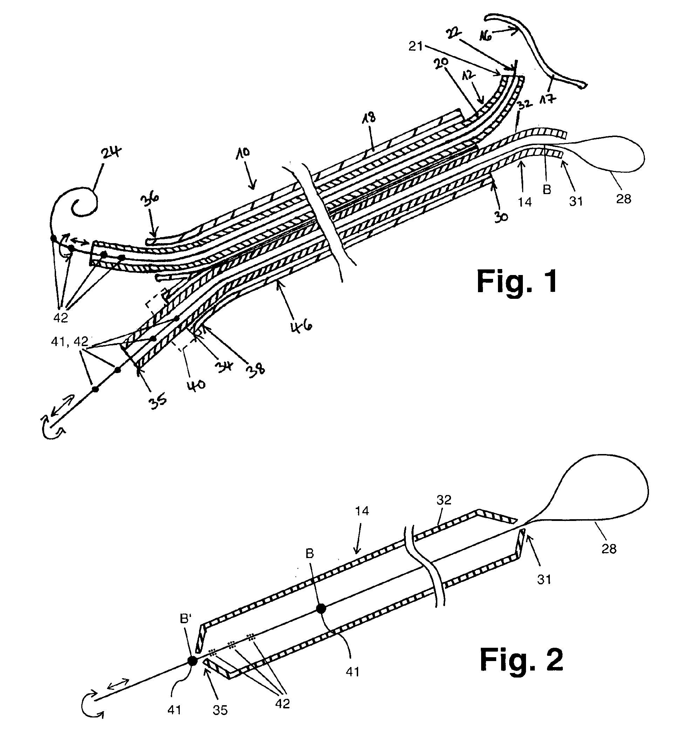

In Fig. 1 a schematic view of a retrieval device 10 according to an embodiment of the present invention is shown.

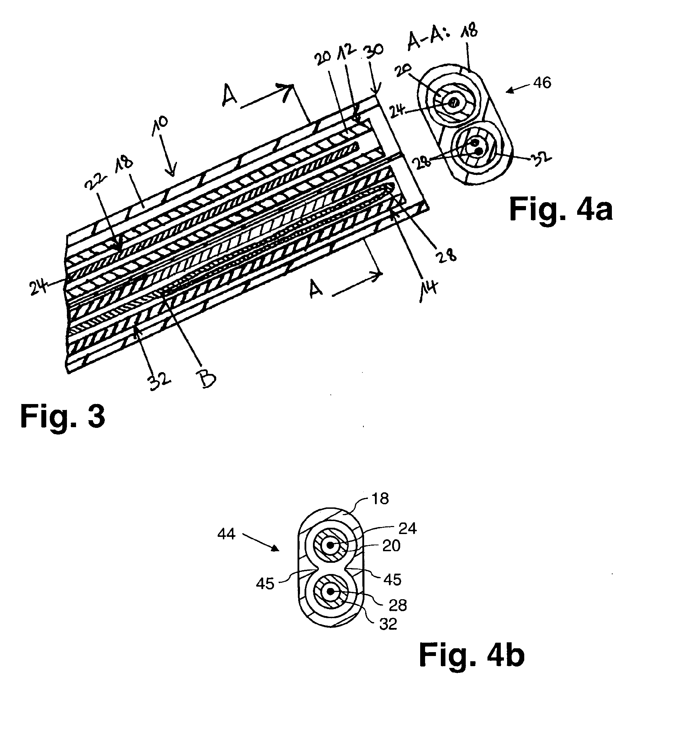

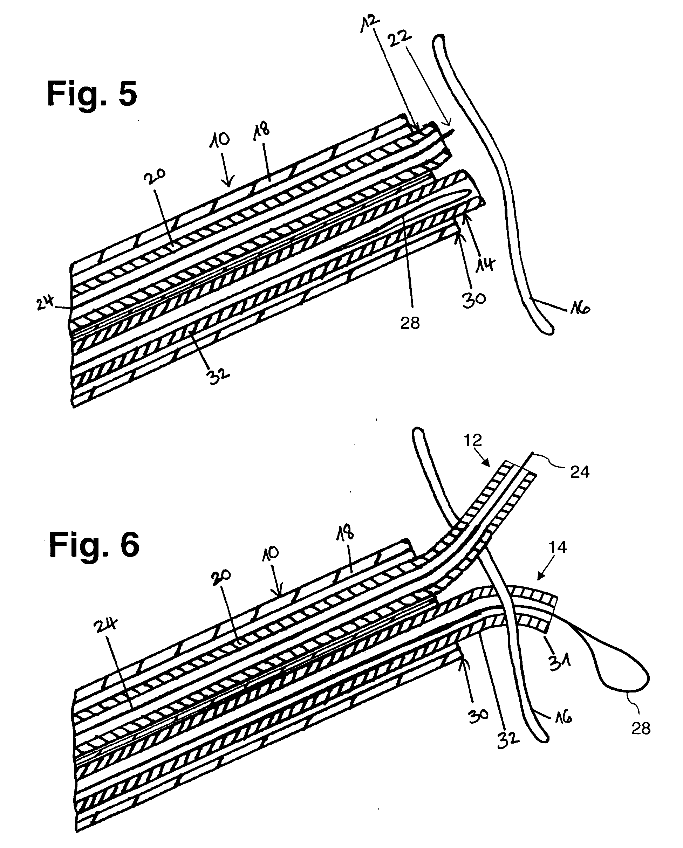

The retrieval device 10 comprises a support member 12 and a connecting member 14, wherein the connecting member 14 can be connected with the support member 12 to enclose a location of an article 16 to be retrieved, as will be shown in further detail, e.g., in Figs. 5-15. In Fig. 1 the support member 12 and the connecting member 14 can be positioned within a transportation or sheath device 18, e.g. a catheter. The transportation or sheath device 18 shown in Fig. 1 is for example a double-lumen or dual-lumen catheter. However, also a single lumen-catheter (see Fig. 4b below), preferably deployed together with a guide rail, or any multi-lumen-catheter etc. can be used or any other sort of catheter which is suitable to house the support member 12 and the connecting member 14 so that they can be rotated independently and can be moved forward and backward separately.

The support member 12 comprises a guide tube 20 in which a guide device 22 is movably located. That means the guide device 22 can be for example moved forward and backward inside the guide tube 20 and further rotated as indicated by the arrows in Fig. 1. The guide device 22 comprises for example at least one flexible guide wire 24. The guide wire 24 can be made of metal and/or of a shape memory material, such as for example Nitinol, or any other shape memory material or shape memory material combination that is flexible and, as the case may be, that is detectable, for instance by means of radioscopic and/or fluoroscopic techniques, in particular by means of X-rays. The guide tube 20 is also flexible and detectable, in particular via X-ray, and can be made of plastic and/or a shape memory material which is flexible, such as Nitinol, or a shape memory material combination which is flexible.

Further, in the embodiment illustrated in Fig. 1, the guide wire 24 is stiffer than the guide tube 20 in which the guide wire 24 is movably arranged. In case the guide wire 24 is stiffer than the guide tube 20, the guide wire 24 can define the form of the guide tube 20, when the guide wire 24 is inserted in or located inside the guide tube 20. As a consequence, when the guide wire 24 is removed from the proximal end of the guide tube 20 by retracting the guide wire 24, the respective part of the guide tube is constructed to form or assume a predefined form or shape, preferably a hook 26 as will be explained in more detail with respect to the following Figs. 5 to 15.

As can be further obtained from Fig. 1, the connecting member 14 comprises a snare 28. The snare 28 is slidably or movably arranged in an additional tube 32, wherein the additional tube 32 is further slidably or movably arranged in the sheath device 18. The snare 28 is folded to be located within the additional tube 32 and can be deployed when moved out of the sheath device 18 and the additional tube 32 as shown in Fig. 1. In an embodiment of the invention, the connecting member 14 can comprise a sheath or multipurpose-catheter, instead of additional tube 32, in which the snare 28 can be slidably or movably located. However, the additional tube 32 or sheath or multipurpose-catheter is an optional feature and can be omitted if the snare 28 is located in the sheath device 18 stable enough to be rotated and moved separately forward and backward, e.g. when the sheath device 18 is a double lumen or a multi-lumen catheter.

The additional tube 32 or sheath is preferably flexible. Furthermore, the additional tube 32 or sheath can be made of any suitable material or material combination (including plastic and/or metal), which is flexible and, as the case may be, detectable, e.g. via X-ray. As shown in Fig. 1, the additional tube 32 or sheath can be located slidably or movably within the sheath device 18. This means the additional tube 32 can be moved forward and backward and further rotated with respect to the sheath device 18. The same also applies to the guide tube 20 with respect to the sheath device 18.

As already mentioned above, the retrieval device 10 or, more specifically, its single components, i.e. the guide tube 20 and the guide device 24 of the supporting member 12 as well as the additional tube 32 and the snare 28 of the connecting member 14 can be made out of any material that is flexible and, as the case may be, that is detectable, for instance by means of radioscopic and/or fluoroscopic techniques, in particular by means of X-rays. The characteristic of being detectable by means of radioscopic and/or fluoroscopic techniques is of importance in particular with respect to application scenarios of the retrieval device 10 inside a human or veterinary patient, in which no other control, e.g. endoscopic control, is available or possible.

For example, according to embodiments of the present invention Multi Purpose Angiographic Catheters from Cordis Corporation, Johnson&Johnson or sundry Guider Catheters from Boston Scientific etc. are appropriate to be employed as additional tube 32 of the connecting member 14 and/or as guide tube 20 of the supporting member 12. An example of a small Pigtail-catheter which can be used in embodiments of the invention, such as a Pigtail-catheter for children, can have a thickness of, e.g., 3 French. As a matter of principle even smaller catheters can be used in embodiments of the invention.

Further, a Snare Kit, Amplatz Goose Neck from Angiotech can be for example used to provide the connecting member 14 comprising the snare 28. However, the invention is not limited to these particular examples of catheters and the snare. The above mentioned catheters and the snare are only exemplary.

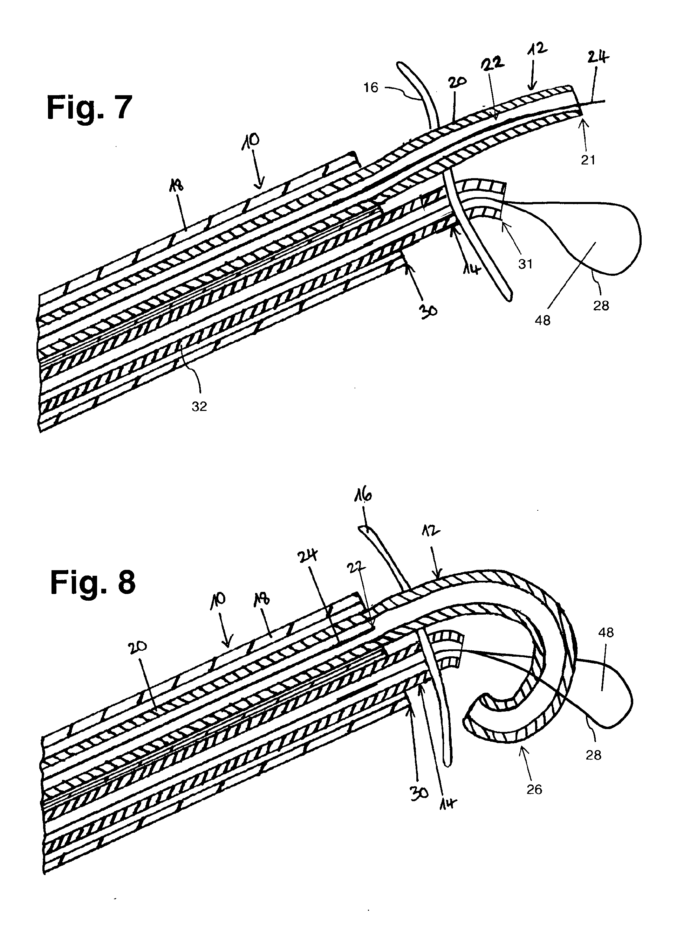

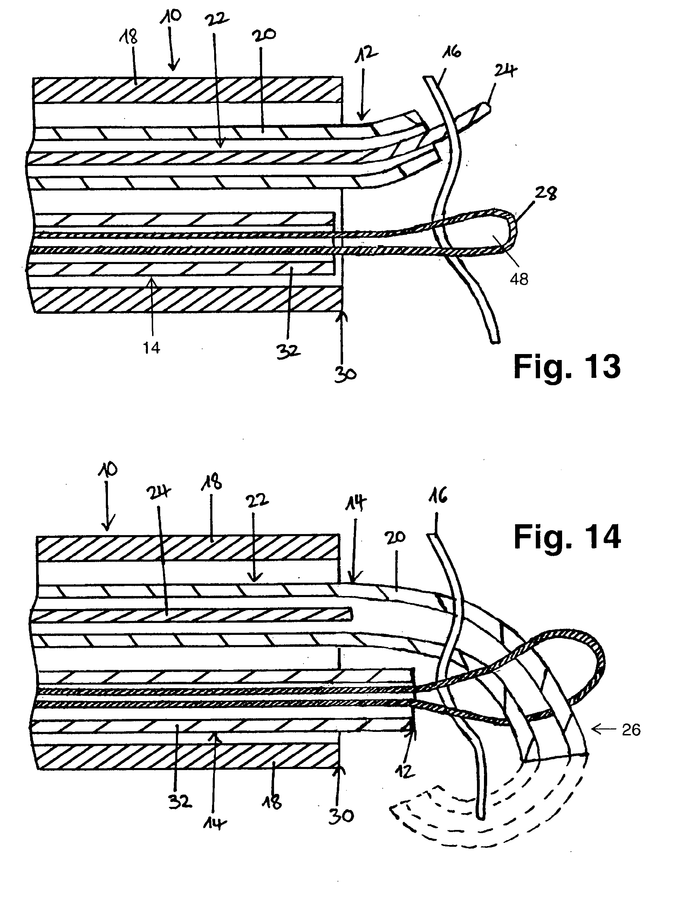

Fig. 1 shows a state of the retrieval device 10 in a basic position in which the support member 12 as well as the connecting member 14 have partially been moved out of the sheath device 18, so that the proximal end 21 of the guide tube 20 and the proximal end 31 of the additional tube 32 of the connecting member 14 protrude from the proximal end 30 of the sheath device 18. The protruding parts of the guide tube 20 (together with the guide device 22 contained therein) as well as of the additional tube 32 of the connecting member 14 assume a slightly bent configuration. Such basic position has proved most suitable for the deployment of the retrieval device 10 with respect to getting the catheter 16 to be retrieved positioned between the supporting member 12 and the connecting member 14, as will be described in more detail in connection with Figs. 6 and 7. Alternatively, as shown in Fig. 14-15 it may be provided that one of the members, i.e. either the supporting member 12, when the guide device 22 is inserted, or the connecting member 14, assumes a basic position that has a straight or essentially straight configuration. If the connecting member 14 is straight or essentially straight, it is provided that the snare 28 and/or hook 26 have a bent configuration.

At the distal end 34 of the sheath device 18 the double-lumen catheter 18 comprises at least one entry or as shown in Fig. 1, e.g., two entries 36, 38, one entry 36 for the support member 12 and one entry 38 for the connecting member 14. The distal end 34 of the sheath device 18 at the entry 38 for the connecting member 14 and/or the entry 36 of the support member 12 can be provided with a fixation means 40. The fixation means 40 is adapted to fix, e.g., the additional tube 32 or snare 28 (in case no additional tube 32 to house the snare 28 is provided) of the connecting member 14 and the guide tube 20 and/or the guide device 22 of the support member 12 in defined positions. In the embodiment of Fig. 1 the fixation means 40 is shown by dashed lines, wherein the fixation means 40 is provided at the entry 38 of the distal end 34 of the sheath device 18 to fix the additional tube 32, for example, in a particular or defined position.

Further, the guide device 22, in the present case the guide wire 24, and/or the snare 28 can be provided with one or a plurality of markers 42. The markers 42 can be provided in the form of swellings 41, such as, e.g., knots, beads or knobs, in form of coloured marks, and/or in form of marks being detectable via X-rays. For instance, a number of markers 42 can be disposed along the distal end of the guide wire 24 and the snare 28, respectively, with equidistance there between. Such markers 42 may assist a user of the retrieval device 10 in determining and controlling the protrusion of the guide wire 24 with respect to the proximal end 21 of the guide tube 20 and of the snare 28 with respect to the proximal end 31 of the additional tube 32, respectively, in an accurate fashion. Additionally or alternatively, markers (not shown in the Figs.) may be provided on the peripheral surface of the distal end of the guide tube 20 and the distal end 35 of the additional tube 32, respectively. Such markers may assist a user of the retrieval device 10 in determining and controlling the rotational position of the guide tube 20 and the additional tube 32, respectively, with respect to the sheath device 18 and/or with respect to each other in an accurate fashion.

The fixation means 40 and the markers 42 can be provided in each embodiment of the inventive retrieval 10 device. However, the features which relates to the fixation means 40 and the markers 42 are optional features and can be also omitted.

Furthermore, the snare 28 can be provided by at least one wire, fibre and/or yarn, wherein the wire, fibre and/or yarn can be made out of or can comprise, e.g., metal and/or a plastic material etc. Preferably, the snare 28 is flexible but also provides a sufficient stiffness to deploy the snare 28. That means the snare 28 is on the one hand sufficiently flexible, so that the snare 28 can be folded or compressed for inserting the snare 28 into the sheath device 18 or into the additional tube 32. On the other hand the snare 28 comprises a sufficient stiffness, so that the snare 28 can be unfolded or deployed when the snare 28 is moved out of the sheath device 18 or the additional tube 32 to form an opening or loop.

The wire, fibre and/or yarn can form a closed snare 28, which is fixed or connected at a point B as shown in Figs. 1-12. In a further alternative the wire, fibre and/or yarn can also form an open snare 28 as shown in Figs. 13-15, which has two ends that are not interconnected.

Fig. 2 illustrates a connecting member 14 of a retrieval device 10 according to another embodiment of the present invention. Just as in the embodiment of Fig. 1, the connecting member 14 includes an additional tube 32, in which the snare 28 is movably located. The snare 28 is fixed to form a closed snare 28. The snare 28 can be moved inside the additional tube 32 forward and backward and can be further preferably rotated as indicated by the arrows in Fig. 2.

In Fig. 2, the additional tube 32 of the connecting member 14 comprises a proximal end 31 and a distal end 35. The opening of distal end 35 of the additional tube 32 is reduced in diameter. In the embodiment of Fig. 2, the opening of proximal end 31 of the additional tube 32 is also reduced in diameter. In particular, the diameter of the distal end 35 is smaller than the diameter of swellings 41 disposed on the snare 28 at points B and B'. The swellings 41 prevent the snare 28 both to be moved too far out of the additional tube 32 into a proximal direction and to be moved too far into the additional tube 32 into a distal direction. More specifically, swelling 41 at point B, which is located inside additional tube 32, will prevent further movement of the snare 28 into a distal direction, when it abuts from inside against distal end 35. On the other hand, swelling 41 at point B', which is located outside additional tube 32, will prevent further movement of the snare 28 into a proximal direction, when it abuts from outside against distal end 35.

In addition, as described in connection with Fig. 1, markers 42 for controlling/determining the exact position of snare 28 during intervention can be disposed on snare 28, e.g. at positions between points B and B'. The markers 42 can be provided, e.g., in form of coloured spots and/or marks being detectable via X-ray, such that they are dimensioned small enough to pass through the reduced diameter of distal end 35 of the additional tube 32.

In Fig. 3 a sectional view of a part of an inventive retrieval device 10 is shown and further in Fig. 4a a cross section A-A of the retrieval device 10 of Fig. 3. The sheath device 18 houses the support member 12, which means the guide tube 20 and the guide device 22 or in this case guide wire 24 as shown in Figs. 3 and 4a. Further, the sheath device 18 houses the connecting member 14 comprising a snare 28, in the present case a closed snare 28 which is closed at point B and which is movably inserted in an additional tube 32. The snare 28 is preferably deployable or can be unfolded when moved out of the additional tube 32 and the sheath device 18 as shown, e.g., in following Figs. 6-12.

The retrieval device 10 shown in Figs. 3 is in a folded state or transportation state. In this folded or transportation state the sheath device 18 can be inserted for example in a vessel or another orifice in a human or veterinary patient and moved to an article 16 to be retrieved from the patient. As will be explained in further detail with respect to Figs. 5-12, when the retrieval device 10 reaches the article 16 to be retrieved inside the body the support member 12 and the connecting member 14 can be moved out of the sheath device 18 to capture the article 16 and to remove it from the patient.

As shown in Figs. 3 and 4a the sheath device 18 can be, e.g., a double-lumen catheter 46. A double-lumen catheter proves to be most suitable, since the support member 12 and the connecting member 14 are housed in different lumina of the catheter so that they are completely separated from each other. As a result, any mutual disturbance between the support member 12 and the connecting member 14 is avoided, thereby drastically reducing the risk of dysfunction or failure of the retrieval device 10.

However, as shown in Fig. 4b, which is a cross-section corresponding to the one of Fig. 4a and which relates to an alternative embodiment of an inventive retrieval device 10, the sheath device 18 can be also a single-lumen catheter 44. Preferably, the single-lumen catheter 44 comprises additional guide rails 45 that partially separate the two lumina from each other in order to reduce the risk of mutual influence of the support member 12 and the connecting member 14.

The single-lumen catheter 44 with guide rails 45 and the double-lumen catheter 46 shown in Figs. 4a and 4b are only exemplary and other catheters which have a different cross-section than the catheters shown in Fig. 4a or 4b can be used as well. The invention is not restricted to the catheters and their cross-sections shown in Figs. 4a and 4b. This applies to all embodiments of the invention.

In the following Figs. 5 to 12 one example for retrieving an article 16 or foreign body by the retrieval device 10 as disclosed in Fig. 1 is shown. However, the invention is not restricted to the steps shown in Figs. 5 to 12. In particular, there are many ways to use the inventive retrieval device 10 to remove an article 16 or foreign body from a human or veterinary patient. The use of the inventive retrieval device 10 is not restricted to the particular steps shown in the example in Figs. 5 to 12 and further in Figs. 13 to 15.

Fig. 5 shows a first step to retrieve an article 16, such as for example a catheter or a piece of a catheter, from a human patient. First of all, the sheath device 18 comprising the support member 12 and the connecting member 14 is moved inside the human patient until the catheter 16 to be retrieved is reached. During transport of the supporting member 12 and the connecting member 14 to the catheter 16 to be retrieved they can be located or housed completely inside the sheath device 18 as shown before, e.g., in Fig. 3, or can be moved to some small extend out of the sheath device 18 as shown, e.g., in Fig. 5.

When the sheath device 18 has reached the catheter 16 to be retrieved within the human patient the support member 12 comprising the guide tube 20 and the guide device 22 and further the connecting member 14 comprising the additional tube 32 and the snare 28 are moved out of the sheath device 18 to capture the catheter 16.

As shown in Fig. 6 the support member 12 and the connecting member 14 can be moved so that the catheter 16 is positioned or lies between the guide tube 20 of the support member 12 and the additional tube 32 of the connecting member 14. To facilitate such positioning, it may be provided that the additional tube 32 of the connecting member 14, when being moved out of the sheath device 18, assumes a slightly bent or arcuate form, e.g. a convex form. The same may apply for the support member 12, i.e. as long as the guide wire 24 (i.e. guide device 22) is located within its guide tube 20, the part of the guide tube 20 moved out of the sheath device 18 provides or assumes a slightly bent form, e.g. a convex form. By rotating the guide tube 20 and the additional tube 32, a situation can be realized in which the guide tube 20 and the additional tube 32 are directed away from each other, as shown in Fig. 6. The rotation may be controlled by using the above mentioned markers disposed on the outer peripheral surface of the tubes 20, 32. When the guide tube 20 and the additional tube 32 are directed away from each other, a relatively large spacing exists there between, making it particularly easy to bring the catheter 16 to be retrieved into the desired position between the guide tube 20 and the additional tube 32.

Further, as also shown in Fig. 6, the snare 28 can be moved out of its additional tube 32 so that the snare 28 can be deployed or unfolded. Since in Fig. 6 the guide tube 20 of the support member 12 comprising the guide wire 24 is directed away from the catheter 16, the guide tube 20 and its guide wire 24 can be rotated in a direction towards the catheter 16 and further towards the snare 28 of the connecting member 14 as will become clear from next Fig. 7.

In Fig. 7 the catheter 16 to be retrieved is lying between the tubes 20, 32 of the guide wire 24 and the snare 28. To capture the catheter 16, the guide tube 20 of the support member 12 and its guide wire 24 are turned in a direction towards the catheter 16 and in particular to the opening 48 formed by the snare 28 of the connecting member 14 by manually rotating the guide tube 20 at its distal end in the respective direction. Again, this step can be controlled by using the above mentioned markers disposed on the outer peripheral surface of the tubes 20, 32.

As shown in Fig. 8, to capture and secure the catheter 16 to be retrieved, the guide wire 24 of the support member 12 is moved backward or in direction to the distal end 34 of the sheath device 18. This results in that the guide tube 20 of the support member 12 forms or assumes a predefined form, namely the form of a hook 26 or loop. For instance, this may be achieved by the guide tube 20 being made from or comprising a shape memory material, in particular Nitinol. Hence, the part of the guide tube 20, from which the guide wire 24 is retracted or removed, starts to adapt its original form, which may be the form of a hook 26 or loop. As illustrated in Fig. 8, the rotational position of the guide tube 20 is adjusted in such a way that the hook 26 or loop builds up in a direction towards the snare 28 of the connecting member 24. As a result, during the formation of the hook 26 or loop caused by the retraction of the guide wire 24 from the proximal end part of the guide tube 20, the guide tube 20 encloses the catheter 16 more and more and further passes through the opening 48 formed by the snare 28. Thus, a location of the catheter 16 is fully enclosed by the support member 12 and the connecting member 14 and their tubes 20, 32 and the snare 28. The retrieval device 10 of the invention has the advantage that the article 16 to be retrieved, e.g., a catheter 16 or a piece of a catheter 16, can be captured and secured to the retrieval device 10 at each location of the article, for example at the end or ends of the article 16 or at any other location other than the end or ends of the article.

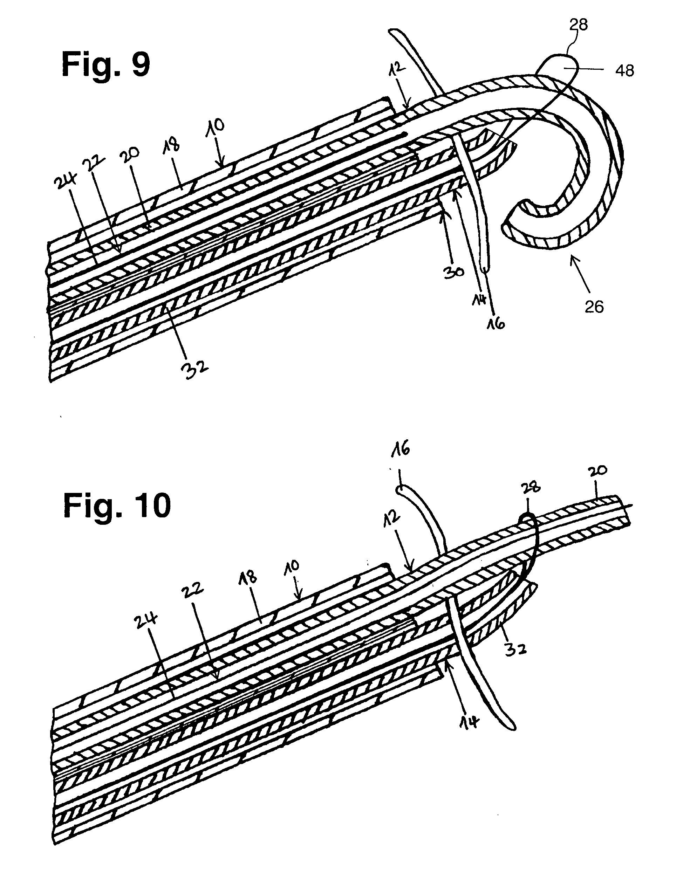

As shown in Fig. 9, to further secure the catheter 16 to be retrieved, so that the catheter 16 can be reliably removed from the patient, the connecting member 14 can be tightly connected to the support member 12. To tightly connect the connecting member 14 to the support member 12, the additional tube 32 is rotated so that the support member 12 and the connecting member 14 are directed to each other. Then the snare 28 is moved backward or in direction to the distal end 34 of the sheath device 18. This results in a reduction of the size of the opening 48 of the snare 28 as shown in Fig. 9. Additionally, the guide tube 20 may be slightly moved forward, i.e. in a proximal direction, in order to avoid a slip off of the snare 28 from the guide tube 20.

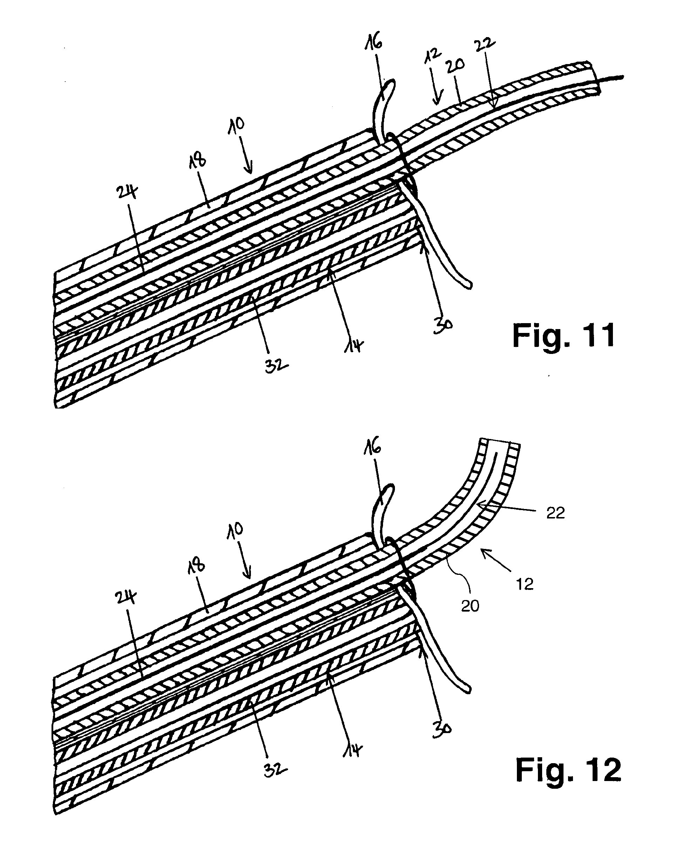

As shown in Fig. 10, to further facilitate connecting the support member 12 and the connecting member 14 and the catheter 16 in between, the guide wire 24 of the support member 12 is moved forward in direction to the proximal end 30 of the sheath device 18 or the guide tube 20 of the support member 12. This results in that the guide tube 20 of the support member 12 forms or assumes its original form or shape, which - basically - is a slightly bent form. However, due to the influence of the snare, the forwarding movement of the guide wire 24 will result in the guide tube assuming a straight or essentially straight form or shape, as shown in Fig. 10. This has the advantage that an unintended slipping off of the snare 28 from the guide tube 20 of the support member 12 can be prevented. Further, the guide tube 20 and the guide wire 24 of the support member 12 can be then moved together backward or in direction to the distal end 34 of the sheath device 18 together with the connecting member 14 and its tube 32 and its snare 28. At the end, the catheter 16 abuts against or is pressed against the proximal end portion of the sheath device 18, wherein the catheter 16 is tightly enclosed by the support member 12 and the connecting member 14 as shown in Fig. 11.

In Fig. 11 the guide tube 20 and the guide wire 24 of the support member 12 and the additional tube 32 and the snare 28 of the connecting member 14 are moved backward or in a direction towards the distal end 34 of the sheath device 18 until the catheter 16 is tightly enclosed and captured by the supporting member 12 and the connecting member 14. In this case the catheter 16 abuts against the proximal end of the sheath device 18 but is preferably not drawn not even partially into the sheath device 18 as it is the case in document US 5,562,678 described above with respect to the state of the art. This has the advantage that the dimensions of the sheath device 18, in particular its diameter, can be kept very small, so that the retrieval device 10 can be introduced even in very small vessels. At the same time, by specific construction of the inventive retrieval device 10, the catheter 16 is tightly and securely hold. Even when exerting rather strong forces to retract the retrieval device 10 together with the catheter 16 from the vessel, a slipping off of the catheter 16 is almost impossible.

In a next step, illustrated in Fig. 12, the guide tube 20 of the support member 12 may be rotated, in particular by 180□ or approximately 180□, such that the guide tube 20 is bent outwardly, i.e. away from the connecting member 14. By doing so, a "stripping off" of the retrieved article 16, in particular during its transportation through the vessel, is effectively avoided. In this embodiment the guide tube 20 of the support member 12 has a concave bending directed outward when the guide device 22 is inserted.

The retrieval device 10 and the sheath device 18 are preferably designed, so that the catheter 16 is neither partially nor completely drawn inside the sheath device 18 to fix the catheter 16. Instead, the catheter 16 preferably only abuts against the additional tube 32 of the connecting member 12 and/or the sheath device 18. The fact that the catheter 16 abuts against the sheath device 18 and/or the additional tube 32 of the connecting member 14 but is not drawn inside the sheath device 18 and/or the additional tube 32 has the advantage, that the diameter of sheath device 18 can be designed relatively small so that the sheath device 18 and, thus, the inventive retrieval device 10 can be used even in very small vessels or orifices of a body.

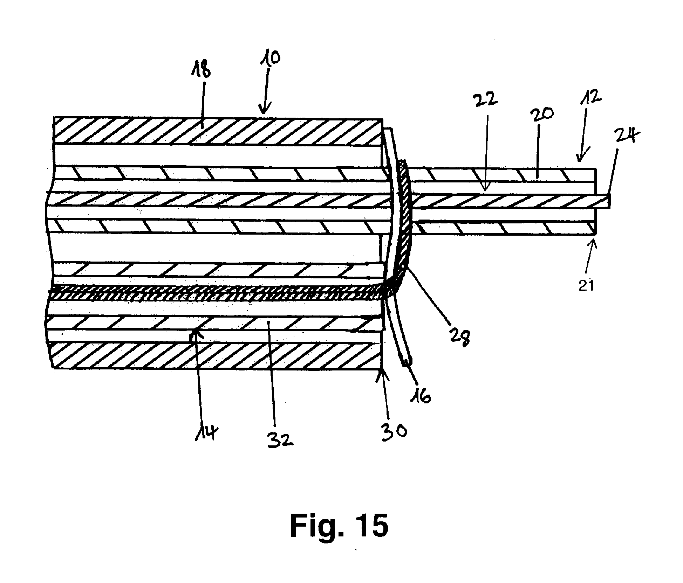

Fig. 13 shows a sectional view of the proximal end of a further embodiment of the inventive retrieval device 10. The example shown in Fig. 13 and following Figs. 14 and 15 differs from the example of the embodiment of the retrieval device 10 shown, e.g., in Figs. 1 to 4 in that the sheath device 18 comprises a single-lumen-catheter and the connecting member 14 comprises an open snare 28 or a snare with an open loop. That means the snare 28 forms an open loop which is not closed at one point B as the snare 28 shown, e.g., in Figs. 1 and 2. A snare 28 comprising an open loop has the advantage that the size of the opening 48 formed by the snare 28 can be varied in a wide range.

In a further embodiment as shown in Fig. 14 the retrieval device 10 comprises as a sheath device 18, for example a single-lumen catheter. The connecting member 14 comprises an open snare 28 or a snare with an open loop. The connecting member 14 has a straight or an essentially straight configuration. The snare and the open loop have a bent or a slightly bent form.

In a further embodiment as shown in Fig. 15 the retrieval device 10 comprises as a sheath device 18 for example a single-lumen catheter. The connecting member 14 comprises an open snare 28 or a snare with an open loop. The support member 12 has a straight or an essentially straight configuration when the guide device 22 is inserted.

Inside the sheath device 18 a connecting member 14 comprising an additional tube 32 and the snare 28 are movably located. The snare 28 is further movably located inside the additional tube 32. The sheath device 18 further comprises a support member 12 comprising a guide tube 20 movably located inside the sheath device 18. Inside the guide tube 20 a guide device 24 is movably located. This applies to all embodiments of the invention. To capture an article 16 to be retrieved, e.g. a catheter 16 as shown in Fig. 13, the support member 12 is moved beneath the catheter 16 and the connecting member 14 is moved above the catheter 16 or vice versa.

In the embodiment of the retrieval device 10 shown in Figs. 13-15, the guide tube 20 and the guide device 22 of the support member 12 are designed, so that when the support member 12 is moved at least partially out of the sheath device 18, the guide tube 20 forms a first predefined configuration, e.g., a slightly bent configuration, in an area in which the guide device 22 is located inside the guide tube 20 (see Fig. 13) and further the guide tube 20 forms a second predefined configuration, i.e. a hook 26, in the area in which the guide device 22 is not located inside the guide tube 20 (see Fig. 14). In other words, when the support member 12 is moved at least partially out of the sheath device 18 and the guide device 22 is moved backward or in a direction towards the distal end 34 of the sheath device 18, the guide tube 20 starts to form a hook 26 in the area of the guide tube 20, from which the guide device 22 is retracted, as shown in Fig. 14.

In Fig. 14 a further step is shown to retrieve the catheter 16 by the retrieval device 10. To fully enclose the catheter 16 by the support member 12 and the connecting member 14 or in other words to connect the connecting member 14 and the support member 12 while the catheter 16 lies between said two members 12, 14, the guide wire 24 is moved backward inside the guide tube 20. This results in that the guide tube 20 outside the sheath device 18 starts to form or to assume a hook 26. As shown in Fig. 14, the guide tube 20 can be designed so that the hook 26 formed by the guide tube 20 is bent only upward and downward, respectively. Further, as shown by the dashed lines in Fig. 14 the guide tube 20 can be designed so that the hook 26 formed by the guide tube 20 can be bent not only upward or downward but also backwards, so that it can form or assume even a loop or loops.

To secure the catheter 16 to be retrieved from the body of the patient, the support member 12 and the connecting member 14 can be moved, so that the catheter 16 is preferably firmly fixed, as shown in the embodiment of Fig. 15.

In the example shown in Fig. 15, the guide wire 24 has been moved forward or, more precisely, into a direction towards the proximal end 21 of the guide tube 20, resulting in that the form or shape of the guide tube returned to its first predefined configuration, which might be a slightly bent configuration or, as illustrated in Fig. 15, an essentially straight configuration. Furthermore, the snare 28 of the connecting member 14 has been moved backward inside its additional tube 32, wherein the additional tube 32 can be also moved backward inside the sheath device 18 together with the snare 28. Further, the guide tube 20 can be moved backward together with the connecting member 14 as well. However, the guide tube 20 should protrude over the proximal end 30 of the sheath device 18 at least to a certain extend, in order to avoid that the snare 28 slips off from the guide tube 20. The exact positioning of the guide tube 20 with respect to the sheath device 18 can again be controlled by using the markers 42 discussed in connection with Fig. 2. At the end the catheter 16 can be firmly hold by the connecting member 14 and its snare 28, respectively, and the guide tube 20, wherein the catheter 16 can abut against the support member 12 and the sheath device 18.

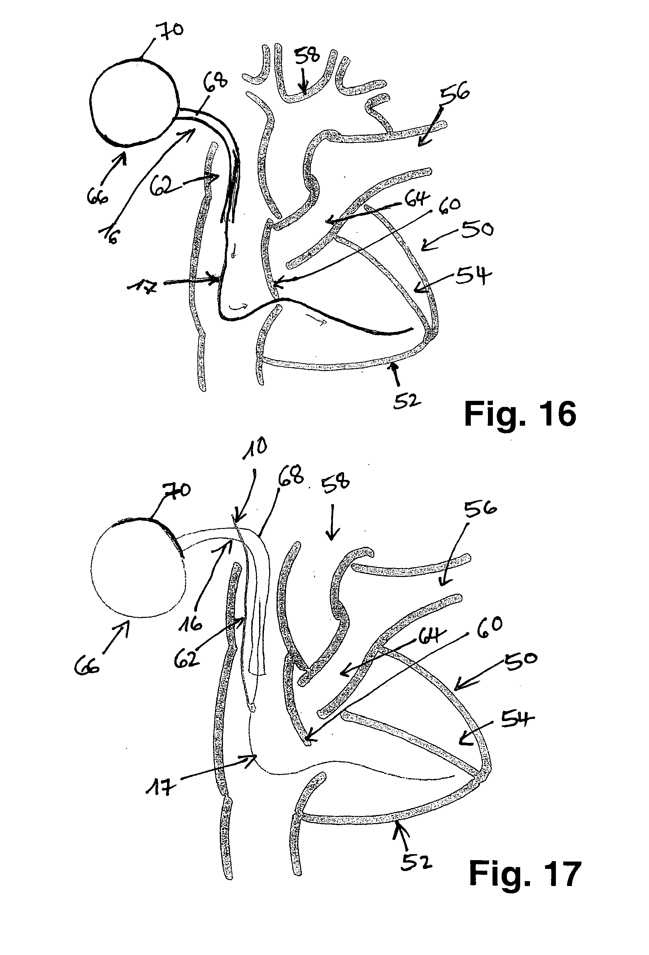

In Fig. 16 a first example is shown of an article 16, e.g. a port-catheter, which is lost in the body of a human patient and has to be retrieved. Fig. 16 shows a simplified and schematic cross-section of a human heart 50. In particular, Fig. 16 shows the right ventricle 52 and the left ventricle 54, the left pulmonary artery 56, the aorta 58, the atrioventricular (tricuspid) valve 60, the superior vena cava 62 and the main pulmonary artery 64.

As can be derived from Fig. 16 a catheter 16, i.e. a port-catheter, has been introduced in the body of a human patient. However, a part 17 of the port-catheter 16 has been disconnected from its port chamber 70 and thus got lost in the patient body. Due to the blood flow the lost part of the port-catheter 17 is transported, e.g., in direction to the heart 50 and the pulmonary artery of the patient. This, however, can cause serious problems and therefore said article 17 has to be retrieved. In the case shown in Fig. 16 for example the lost part 17 of the port-catheter 16 is going to pass the Atrioventricular (tricuspid) valve 60 of the heart 50. In time connective tissue encapsulates every foreign material inside the body. If disconnection of the port catheter 16 from the port chamber 70 does not occur immediately after implantation of the port system 66, connective tissue 68 also starts to encapsulate the proximal part of the port-catheter 16 which is in this way still attached to the port chamber 70 of the port-system 66.

Fig. 17 now shows the retrieval of the disconnected port-catheter 17 with a retrieval device 10 according to the invention, for example the retrieval device 10 shown in Figs. 1 to 3. Fig. 17 shows the heart 50 as shown in Fig. 16 including the port-catheter 16 and the lost part 17 of the port-catheter 16. Further, the retrieval device 10 according to the invention is introduced into the body, e.g., through a vessel of the patient e.g. the internal jugular vein and the retrieval device 10 is transported by a transportation device or sheath device until it reaches a part of the disconnected port-catheter 17. As described before, the inventive retrieval device 10 has the advantage that the retrieval device 10 can capture and retrieve an article 16, 17 to be retrieved at any location of the article 16, 17, for example at the end or ends of the article 16, 17 or at any other location other than the end or ends of the article 16, 17. That means the inventive retrieval device 10 can capture or engage the lost part 17 of the catheter 16 shown in Fig. 17 at any location. The inventive retrieval device 10 can capture the lost part 17 of the catheter 16 even at a location away from the ends of the part of the catheter 16, e.g., at a location in the middle of the catheter 16 and then retrieve the catheter 16. As shown in the example in Fig. 17, the inventive retrieval device 10 can capture or engage the catheter 16 in any area of the catheter 16 which is not encapsulated by connective tissue 68. Examples to operate an inventive retrieval device 10 to capture and retrieve the article 16, 17 to be retrieved, in the present case the lost port-catheter, are shown in Figs. 5 to 15 above.

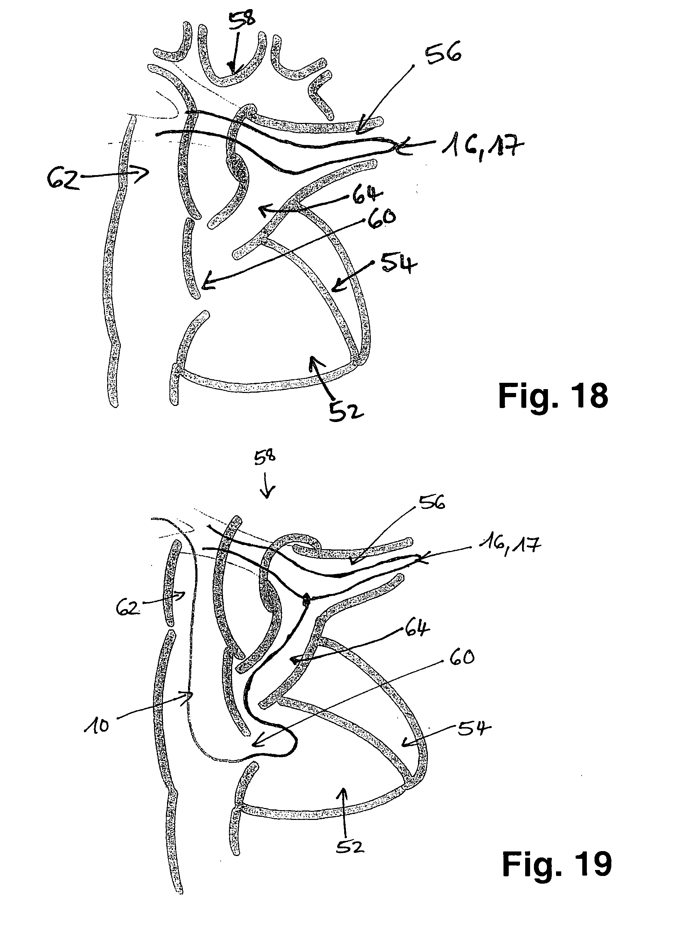

Further, in Fig. 18 a second example is shown of an article 16, e.g. a port-catheter 16, which is lost in the body of a human patient and has to be retrieved. As well as Figs. 16 and 17, Fig. 18 shows a simplified and schematic cross-section of a human heart 50. In particular, Fig. 18 shows the right ventricle 52 and the left ventricle 54, the left pulmonary artery 56, the aorta 58, the atrioventricular (tricuspid) valve 60, the superior vena cava 62 and the main pulmonary artery 64.

As shown in Fig. 18 a port-catheter 16 introduced in the body of a human patient got lost. Due to the blood flow the lost port-catheter 16 is transported, e.g., in direction to the heart 50 and the lung of the patient. In the present case the lost catheter 16 is located inside the left pulmonary artery 56.

In Fig. 19 it is shown how the lost port-catheter 16 can be retrieved with a retrieval device 10 according to the invention. Fig. 19 shows the heart 50 as shown in Fig. 18 including the lost port-catheter 16. To retrieve the catheter 16, the retrieval device 10 according to the invention is introduced into the body, e.g., through the internal jugular vein of the patient or another orifice of the body and the retrieval device 10 is transported via a transportation device or sheath device until it reaches the lost port-catheter 16 in the left pulmonary artery 56 or right pulmonary artery or in the main pulmonary artery 64. As shown in Fig. 19 the inventive retrieval device 10 can capture or engage the lost catheter 16 at any location. In particular, the inventive retrieval device 10 can capture and secure or fix the catheter 16 even at a location away from the ends of the catheter 16, e.g., at a location in the middle of the catheter 16 as shown in the example in Fig. 19, and then retrieve the catheter 16. As described before with respect to Figs. 16 and 17, examples to operate an inventive retrieval device 10 to capture and retrieve the article 16 to be retrieved are shown in Figs. 5 to 15 above.

While this invention has been particularly shown and described with reference to preferred embodiments thereof, it will be understood by those skilled in the art that various changes in form, modification, variation and details may be made therein without departing from the scope of the invention as defined by the appended claims. Further, the embodiments described before with respect to Figs 1 to 15 can be combined, in particular single features of the different embodiments.

### List of reference signs

- 10: retrieval device
- 12: support member
- 14: connecting member
- 16: article to be retrieved, e.g. catheter
- 17: part of a catheter lost in a body of a patient
- 18: transportation device/sheath device
- 20: guide tube
- 21: proximal end (of guide tube 20)
- 22: guide device
- 24: guide wire
- 26: hook
- 28: snare
- 30: proximal end (of transportation device 18)
- 31: proximal end (of additional tube 32)
- 32: additional tube
- 34: distal end (of transportation device 18)
- 35: distal end (of additional tube 32)
- 36: entry (support member)
- 38: entry (connecting member)
- 40: fixation means
- 41: swelling
- 42: marker
- 44: single-lumen catheter
- 45: guide rail
- 46: double-lumen catheter
- 48: opening of the snare
- 50: heart
- 52: right ventricle
- 54: left ventricle
- 56: left pulmonary artery
- 58: aorta
- 60: tricuspid valve
- 62: superior vena cava
- 64: main pulmonary artery
- 66: port-system
- 68: connective tissue
- 70: port chamber

## Claims

1. Retrieval device (10) for retrieving articles comprising:
a support member (12) comprising a guide tube (20) and a guide device (22) which is located movably inside the guide tube (20), and
a connecting member (14) comprising a snare (28),
wherein the connecting member (14) and the support member (12) are used together to capture an article or foreign body to be retrieved.

2. Retrieval device according to claim 1,
**characterized in that,**
the support member (12) is designed or built to get connected with the connecting member (14) to retrieve an article to be retrieved.

3. Retrieval device according to claim 1 or 2,
**characterized in that,**
the support member (12) is designed to be varied between a first form or configuration and a second form or configuration, wherein the first form or configuration is preferably a slightly bent form or configuration and wherein the second form or configuration is preferably a hook form or hook configuration.

4. Retrieval device according to any of claims 1 to 3,
**characterized in that,**
the guide device (22) of the support member (12) comprises a guide wire (24) which is movably located in the guide tube (20).

5. Retrieval device according to any of claims 1 to 4,
**characterized in that,**
the guide tube (20) and the guide device (22) are designed or built, so that at least a part of the guide tube (20) forms or assumes a first predefined form or configuration, preferably a slightly bent form or configuration, when the guide device (22) is located inside this part of the guide tube (20) and further
the guide tube (20) and the guide device (22) are designed or built, so that at least a part of the guide tube (20) forms or assumes a second predefined form or configuration, preferably a hook form or hook configuration, when the guide device (22) is not located inside or is removed and/or retracted from this part of the guide tube (20).

6. Retrieval device according to any of claims 1 to 5,
**characterized in that,**
the connecting member (14) comprises a tube (32) in which the snare (28) is movably located.

7. Retrieval device according to any of claims 1 to 6,
**characterized in that,**
the guide device (22), the guide tube (20), the snare (28) and/or the tube (32) of the connecting member (14)are flexible and are made in particular from plastic and/or metal.

8. Retrieval device according to any of claims 1 to 7,
**characterized in that,**
the guide device (22), the guide tube (20), the snare (28) and/or the tube (32) of the connecting member (14)comprise or are made from a shape memory material, in particular Nitinol.

9. Retrieval device according to any of claims 1 to 8,
**characterized in that,**
the guide device (22), the guide tube (20), the snare (28) and/or the tube (32) of the connecting member (14)comprise or are made from a material, which is detectable by means of fluoroscopic and/or radioscopic techniques, in particular by means of X-rays.

10. Retrieval device according to any of claims 1 to 9,
**characterized in that,**
the guide device (22) and/or the snare (28) comprise at least one wire, fibre and/or yarn.

11. Retrieval device according to any of claims 1 to 10,
**characterized in that,**
the snare (28) is a closed snare (28) or an open snare (28).

12. Retrieval device according to any of claims 1 to 11,
**characterized in that,**
the support member (12) and the connecting member (14) are movably housed in a transportation device (18), wherein the transportation device (18) is preferably a sheath device (18), in particular a single-lumen catheter (44) or a double-lumen catheter (46).

13. Retrieval device according to any of claims 1 to 12,
**characterized in that,**
the guide wire (24) and/or the snare (28) comprise at least one marker (42) to indicate the longitudinal position of the guide wire (24) and the snare (28), respectively, wherein the marker (42) is a swelling, a coloured mark and/or a mark being detectable via fluoroscopic and/or radioscopic techniques, in particular via X-rays.

14. Retrieval device according to any of claims 1 to 13,
**characterized in that,**
that a diameter of an opening of least one end (30, 31, 34, 35) of the guide tube (20) or of the tube (32) of the connecting member (14) is smaller than the diameter of the corresponding marker (42) of the guide wire (24) and the snare (28), respectively.

15. Retrieval device according to any of claims 1 to 14,
**characterized in that,**
at least one marker is provided on the peripheral surface of the guide tube (20) and/or the tube (32) of the connecting member (14), respectively, to indicate the rotational position of the guide tube (20) and the tube (32) of the connecting member (14), respectively, with respect to the transportation device (18) and/or with respect to each other,
and/or
the retrieval device (10) comprises at least one fixation means (40) which is adapted to fix the guide tube (20), the guide wire (24), the tube (32) of the connecting member (14) and/or the snare (28) in a defined position.
